# EUROPÄISCHE PATENTANMELDUNG

(11) **EP 1 465 117 A1**
(43) Veröffentlichungstag der Anmeldung: **06.10.2004**
(21) Anmeldenummer: 03006880.3
(22) Anmeldetag: 31.03.2003
(51) Int. Cl.: G07C 9/00, A61B 5/12

(54) **Verfahren und Vorrichtung zur Personenidentifikation durch Messung otoakustischer Emissionen**

(71) Anmelder: Hotz, Michel André, 2603 Péry (CH); Hauser, Rolf, 79098 Freiburg i.Br. (DE)
(72) Erfinder: Hauser, Rolf, 79098 Freiburg (DE)
(74) Vertreter: Blum, Rudolf Emil Ernst

(57) **Zusammenfassung**

Die Vorrichtung verwendet aktive akustische Aussendungen des Innenohrs zur Überprüfung und/oder Erkennung der Identität einer Person. Wahl und Definition der multiplen Stimulations- und Messparameter gestatten den Aufbau eines programmierbaren Schlüssel-/Schloss-Systems. Dies erlaubt für die zu identifizierende Person eine oder mehrere eindeutige Datensätze zu erzeugen und zur Identitätsprüfung heranzuziehen. Die Stimulations/Messeinheit umfasst Mikrophon (3) und Lautsprecher (2, 24). Die gemessenen Aussendungen werden mit einem Spektrumanalysator in den Frequenzbereich transformiert. Die so gewonnenen individuellen Daten werden mittels Speichermittel (11) gespeichert oder mit einem Vergleichsmittel (12) mit bereits gespeicherten spezifischen Daten verglichen.

## Beschreibung

Die Erfindung betrifft ein Verfahren zur Registrierung und späteren Erkennung und/oder Überprüfung einer Identität eines Individuums gemäss Oberbegriff von Anspruch 1 und eine Vorrichtung zur Ermittlung biometrischer Daten eines Individuums gemäss Oberbegriff von Anspruch 13.

Verfahren und Vorrichtungen dieser Art, wie sie in US 5,787,187 beschrieben sind, werden eingesetzt um biometrische Daten eines Individuums zu ermitteln, insbesondere um so die Identität des Individuums festzustellen.

Generell werden auf dem Gebiet der Biometrie im Wesentlichen unveränderliche Merkmale eines Individuums gemessen und registriert, so dass die Identität des Individuums zu einem späteren Zeitpunkt überprüft oder erkannt werden kann.

Das in US 5,787,187 beschriebene Verfahren misst ein passives akustisches Echo des Gehörgangs. Die auf diese Weise gewonnenen biometrischen Daten haben jedoch nur einen geringen Charakterisierungsgehalt, weshalb die Sicherheit bzw. Zuverlässigkeit des Verfahrens unvollkommen ist.

Bei weiteren bekannten biometrischen Verfahren werden z.B. Fingerabdruck, Iris, Retina, Sprache, Gesicht oder Motorik gemessen. Den meisten dieser Verfahren ist gemeinsam, dass sie während des Messprozesses den Handlungsfreiraum der gemessenen Person signifikant einschränken und eine intellektuelle und/oder physische Aktivität praktisch ausschliessen. Zumindest wird jedoch die gemessene Person abgelenkt oder gestört. So ist es z.B. kaum möglich während einer Irismessung ein Fahrzeug zu steuern. Zudem erreicht bis heute keine der kommerziell erhältlichen, nichtinvasiven biometrischen Technologien akzeptable Fehlerraten. Darüber hinaus lässt die Praktikabilität unter den Bedingungen der Alltagserfordernisse eine breite, effektive und sichere Anwendung aktuell verfügbarer Verfahren nicht zu. Letztlich verhindern eine zu grosse Ähnlichkeit interindividueller Charakteristika und/oder eine hohe intraindividuelle Variabilität biometrischer Messungen einen Durchbruch dieser Verfahren in der Breitenanwendung.

Es stellt sich daher die Aufgabe, ein Verfahren und eine Vorrichtung der eingangs genannten Art bereitzustellen, welches die oben genannten Nachteile zumindest teilweise vermeidet.

Diese Aufgabe wird von den unabhängigen Ansprüchen gelöst, indem die Messung otoakustischer Emissionen zur Identifikation von Personen eingesetzt wird.

Das erfindungsgemässe Verfahren und die erfindungsgemässe Vorrichtung haben den Vorteil, dass sie eine schnelle, zuverlässige, sichere und insbesondere kontinuierliche Bestimmung biometrischer Daten ermöglichen. Das gemessene Individuum kann während der Messung eine Tätigkeit ausführen. Ein weiterer Vorteil liegt darin, dass diese biometrischen Daten eine Schlussfolgerung auf den Vitalzustand des Individuums zulassen.

Weitere Vorteile und bevorzugte Ausführungsbeispiele ergeben sich aus den abhängigen Ansprüchen, sowie aus der nun folgenden Beschreibung anhand der Figuren. Dabei zeigen:
Fig. 1 eine mögliche Messsonde zur Messung otoakustischer Emissionen für die Anwendung im erfindungsgemässen Verfahren,
Fig. 2 ein Flussdiagramm des erfindungsgemässen Verfahrens zur Registrierung und späteren Erkennung und/oder Überprüfung der Identität von Personen,
Fig. 3 ein Blockdiagramm einer Vorrichtung zur Ausführung des erfindungsgemässen Verfahrens,
Fig. 4 Messsignale otoakustischer Emissionen bei unterschiedlichen Stimuluslautstärken,
Fig. 5 Messsignale otoakustischer Emissionen zweier Ohren im Zeit- und Frequenzbereich,
Fig. 6 ein Messsignal mit Stimulus und otoakustischer Antwort im Frequenzbereich,
Fig. 7 ein Anwendungsbeispiel des erfindungsgemässen Verfahrens zur Sicherung eines Tresorraumes.

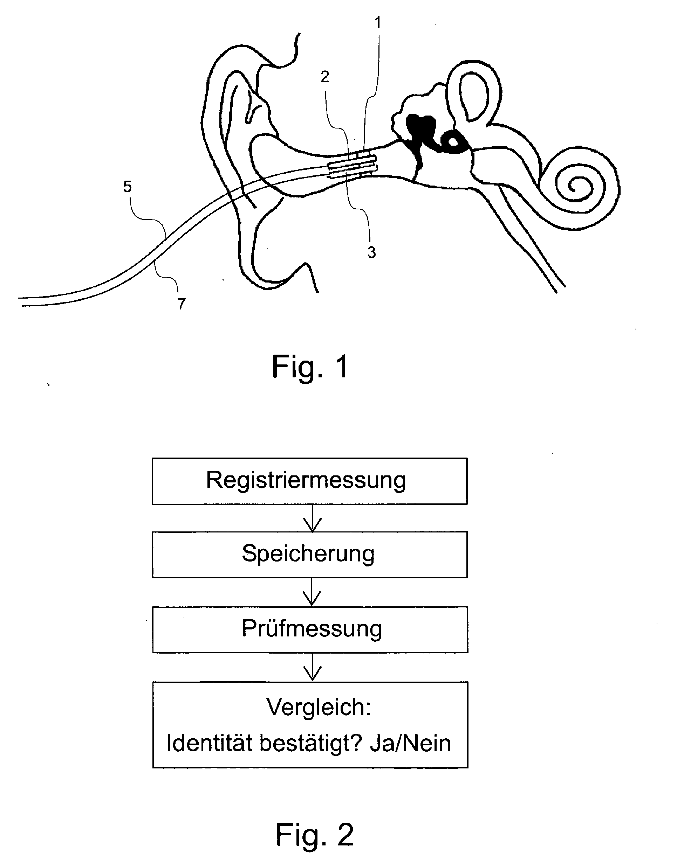

Fig. 1 zeigt eine Messsonde zur Messung otoakustischer Emissionen. Die erfindungsgemässe Vorrichtung zur Ermittlung biometrischer Daten weist bevorzugt eine solche Messsonde auf. Die Messsonde bzw. Gehörgangssonde 1 umfasst einen Lautsprecher 2 und ein Mikrophon 3. Lautsprecher 2 und Mikrophon 3 können auch kombiniert werden, wobei das kombinierte Element insbesondere nur eine einzige Membran aufweisen kann. Stimulussignale werden über ein Lautsprecherkabel 5 und die Mess- bzw. Antwortsignale über ein Mikrophonkabel 7 von bzw. zu einer Messstation bzw. einem Computersystem übertragen. Anstelle einer Gehörgangssonde 1 können für die Messung otoakustischer Signale auch hinter dem Trommelfell angeordnete implantierbare oder teilimplantierbare Messsonden mit kabelloser Übertragungstechnik verwendet werden. Ein Lautsprecher 2 ist insbesondere dann notwendig, wenn akustisch evozierte otoakustische Emissionen gemessen werden sollen. Falls alleine spontane otoakustische Emissionen gemessen werden sollen, ist nicht unbedingt ein Lautsprecher 2 erforderlich. Spontane otoakustische Emissionen können jedoch durch einen akustischen Stimulus synchronisiert werden.

Otoakustische Emissionen sind individuelle aktive cochleäre, d.h. von der Hörschnecke produzierte, akustische Aussendungen der linken und/oder rechten Hörschnecke und werden im Zusammenhang mit der Schallanalyse des Ohres ausgesendet. Sie sind über lange Zeiträume intraindividuell ausserordentlich stabil und reproduzierbar und sind nur beim lebenden Organismus nachweisbar, da sie auf stoffwechselabhängigen und damit energieabhängigen Prozessen der Innenohraktivität basieren. Sie werden aktiv von einem lebenden Innenohr vermutlich im Bereich der äusseren Haarzellen ausgesandt. Die äusseren Haarzellen in der Hörschnecke kontrahieren sich während ihrer Tätigkeit aktiv und senden entweder spontan oder als Antwort auf einen akustischen Stimulus mit einer frequenzspezifischen Verzögerungszeit (Latenzzeit) leise Impulse aus, die mit empfindlichen Mikrofonen gemessen werden können. Jedes Innenohr sendet spontan oder je nach intermittierender oder kontinuierlich applizierter, frequenzabhängiger akustischer Stimulation individuell unterschiedliche Antworten aus, die unter definierten Randbedingungen in Amplitude und Frequenz streng reproduzierbar sind. Unter unterschiedlichen Stimulationsbedingungen werden unterschiedliche, aber höchst reproduzierbare Antworten produziert. Diese "Antworten des Innenohres", die einen aktiven, energieabhängigen Prozess wiederspiegeln, können aufgrund ihrer nichtlinearen Eigenschaften über spezielle Stimulations- und Antwortextraktionsalgorithmen aus Resonanzen und sonstigen passiven Echos des Ohres extrahiert oder zu diesen in Verhältnis gesetzt werden. Otoakustische Emissionen zeichnen sich durch eine Vielzahl von Eigenschaften aus, die ihre individuelle und eindeutige Zuordnung zu bestimmten Personen und prinzipiell auch anderen Lebewesen ermöglichen. Otoakustische Emissionen gehorchen verschiedenen Gesetzmässigkeiten, die es ermöglichen durch die Wahl der Stimulations und/oder Messparameter ein programmierbares Schlüssel-Schloss-System zu schaffen. Für das erfindungsgemässe Verfahren werden sowohl spontane otoakustische Emissionen (SOAE), Stimulusfrequenzemissionen (SFOAE), Transitorisch evozierte otoakustische Emissionen (TEOAE) als auch otoakustische Emissionen cochleärer Distorsionsprodukte (DPOAE) alleine oder in Kombination genutzt, um programmierbare eindeutige Antworten der individuellen Hörschnecke abzugreifen. Die Emissionen können prinzipiell direkt am Innenohr, im Mittelohr oder im äusseren Kompartiment des Ohres gemessen werden. Weitere Eigenschaften und Besonderheiten otoakustischer Emissionen und deren Messung sind beschrieben in: Hauser. R, Anwendung otoakustischer Emissionen. Ein Kompendium für Klinik und Praxis, Stuttgart: Enke-Verlag, 1998; 2. Auflage.

Fig. 2 zeigt die prinzipiellen Schritte des erfindungsgemässen Verfahrens zur Registrierung und späteren Erkennung und/oder Überprüfung der Identität von Individuen. Im Folgenden wird der Begriff "Person" verwendet, was jedoch nicht ausschliessen soll, dass das erfindungsgemässe Verfahren auch für andere Lebewesen bzw. Tiere verwendet werden kann. Beim erfindungsgemässen Verfahren werden zunächst in einer Registriermessung biometrische Daten einer Person ermittelt. Diese Ausgangs-, Basis- bzw. Referenzdaten werden gespeichert, insbesondere zusammen mit weiteren personenbezogenen Daten, wie z.B. dem Namen der Person oder einer Kennzahl.'Es können eine oder aber auch mehrere Personen auf diese Weise registriert werden. Die biometrischen Daten umfassen Information zu otoakustischen Emissionen der jeweiligen Person. Diese Daten bzw. Charakterisierungswerte bilden ein individuelles "Innenohrprofil". Es ist jedoch auch eine Kombination mit weiteren biometrischen Personenmerkmalen, wie z.B. Fingerabdruck oder Sprache und/oder zusätzlichen Passwörtern möglich. Die Messsignale bzw. die biometrischen Daten werden bevorzugt in einer Form gespeichert, welche ein effizientes Vergleichen bzw. Auffinden ähnlicher biometrischer Daten ermöglicht. Insbesondere werden sie dazu aufbereitet und analysiert. So kann z.B. eine Artefaktunterdrückung bzw. Extraktion der otoakustischen Emissionen durchgeführt werden. Analyseparameter können sein: das Frequenzspektrum bzw. das Fourierspektrum, die Amplituden der Antworten, das angewandte Mittelungsverfahren, die Latenzzeiten der Antworten, die Reproduzierbarkeit, Linearitäten, Nicht-linearitäten und die Stabilität des Stimulationssignales, die während der Messung aufgezeichnet werden können. Insbesondere können die Signale für die bzw. bei der Analyse z.B. in einen Frequenzbereich transformiert werden und/oder es wird z.B. eine computer-implementierte Mustererkennung durchgeführt. Die gespeicherten biometrischen Daten weisen insbesondere in einen Frequenzbereich transformierte Signaldaten und/oder in einen Frequenzbereich transformierbare Signaldaten auf. Die biometrischen Daten können zeitweise oder dauernd, innerhalb oder ausserhalb eines Computersystems gespeichert werden.

Zur Erkennung oder Überprüfung der Identität eines Individuums wird zunächst eine Prüfmessung durchgeführt. Die Messparameter sind dabei bevorzugt dieselben wie bei einer Registriermessung. Anschliessend werden die Resultate der Prüfmessung mit den gespeicherten Daten verglichen. Für eine Identitätsüberprüfung reicht im Wesentlichen ein Vergleich, für eine Identitätserkennung sind mehrere derartige Vergleiche erforderlich. Für den Vergleich werden die Messsignale der Prüfmessung aufbereitet und analysiert, im Wesentlichen auf die gleiche Weise wie bereits oben in Bezug auf die Registriermessung beschrieben. Beim Vergleich wird das vom Innenohr emittierte individuelle Datenmaterial bzw. die Innenohrcharakteristik kontinuierlich oder punktuell nach Massgabe individuell wählbarer Parameter- und/oder Entschlüsselungs-Codes mit den Datensätzen registierter Personen verglichen. Die Daten werden insbesondere hinsichtlich der Amplitude, Frequenzverteilung und möglicherweise der frequenzspezifischen Latenzzeiten und/oder anderer Parameter bewertet.

Vorzugsweise ist sowohl bei der Registriermessung als auch bei der Prüfmessung eine automatische Kalibrierung vorgesehen, um störende Einflüsse des Mittelohres und/oder äusseren Ohres und/oder sonstiger Strukturen auf die genuinen, aktiven akustischen Aussendungen des Innenohres zu eliminieren. Dabei kann eine Kalibrierung entsprechend der beabsichtigten Stimulationstechnik zur Schaffung eines zusätzlichen Parameters genutzt werden. Zudem sind Störschallunterdrückungsmechanismen und - algorithmen vom System vorgesehen, z.B. Störschallpegelbegrenzung, alternative Speicherung aufeinanderfolgender Messungen, Produktion von Antischall im tieferen Frequenzbereich.

Das erfindungsgemässe Verfahren ermöglicht nicht nur eine sichere Identifikation der Innenohraktivität eines spezifischen Individuums, sondern auch eine Aussage über den Zustand des geprüften Individuums hinsichtlich seiner Vitalität. So kann aus den Messresultaten der Vitalstatus des Individuums bestimmt werden, da nur beim lebenden Individuum otoakustische Emissionen erwartungsgemäss nachweisbar sind. Dies deshalb, weil die Generierung der Emissionen im Innenohr einen aktiven energieverbrauchenden Prozess voraussetzt. Darüberhinaus können sich die Innenohreigenschaften in Form der otoakustischen Emissionen durch die Einnahme von Drogen verändern, so dass ein Drogenmonitoring, z.B. hinsichtlich Alkohol, möglich ist. Das erfindungsgemässe Verfahren hat den Vorteil, dass die biometrische Messung selbst sehr einfach durchzuführen ist. Sie ist nichtinvasiv und somit wenig belastend, so dass sie von der zu prüfenden Person problemlos akzeptiert wird und sogar bei Neugeboren durchgeführt werden kann. Keine für die breite Anwendung unpraktikable Belastung oder Belästigung oder invasive, unkomfortable Prozeduren wie z.B. ein Abtasten der Retina oder Iris des Auges, des Fingerabdrucks oder der Stimmenidentifikation sind notwendig.

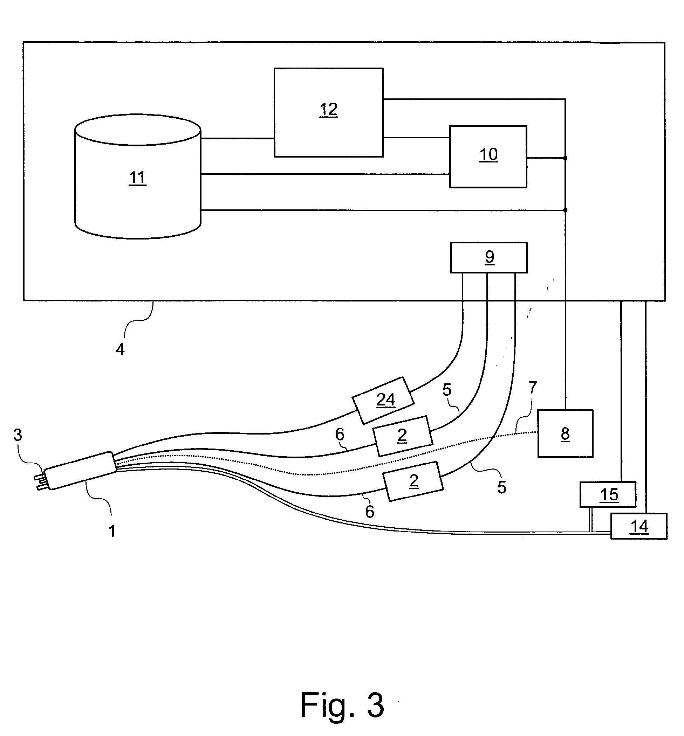

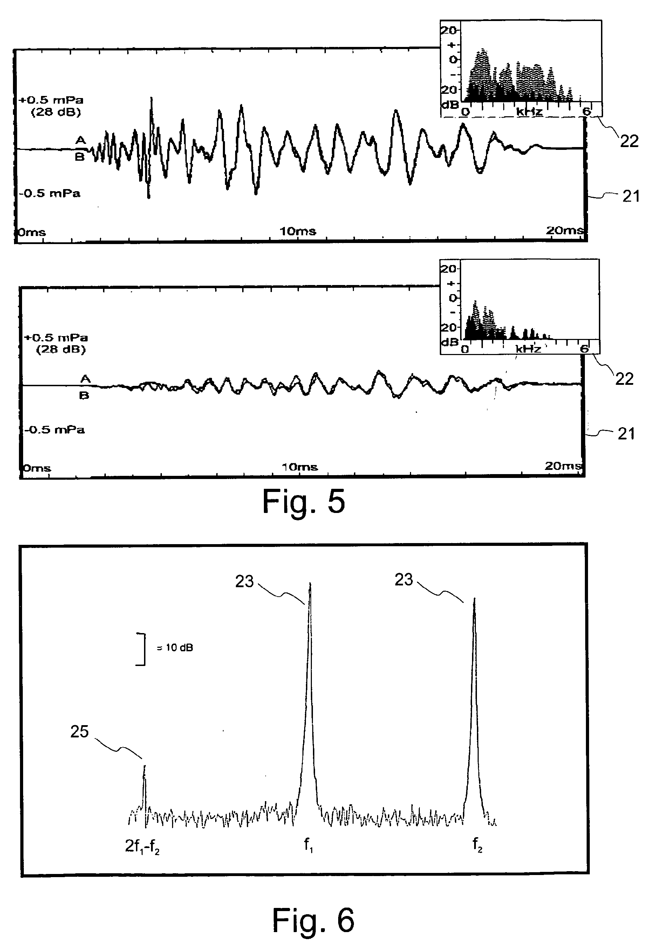

Fig. 3 zeigt ein Blockdiagramm einer Vorrichtung bzw. eines Systems zur Ausführung des erfindungsgemässen Verfahrens zur Registrierung und späteren Überprüfung und/oder Erkennung der Identität eines Individuums mittels Messung otoakustischer Emissionen. Die Vorrichtung weist ein Computersystem 4 mit Steuerungs- und Analyse-Software auf. In einer bevorzugten Ausführung ist das Computersystem 4 ein PC, d.h. Personal Computer. Dies hat den Vorteil, dass die Vorrichtung kostengünstiger ist, weil so bezüglich des Computersystems in den meisten Fällen auf eine bereits vorhandene Hardware zurückgegriffen werden kann. In einer weiteren bevorzugten Ausführung besteht das Computersystem 4 im Wesentlichen aus einem Microcontroller und/oder einem Signalprozessor, was wegen des geringen Stromverbrauches und der kleinen Abmessungen speziell für tragbare Ausführungen der Vorrichtung vorteilhaft ist. Die Vorrichtung weist ferner eine Gehörgangssonde 1 mit einem sensitiven Mikrophon 3 auf. Das Mikrophon 3 ist über ein Kabel 7 mit einem Verstärker 8 verbunden. Der Verstärker 8 weist bevorzugt einen Hochpassfilter mit einer Grenzfrequenz von ca. 400 Hz und einen Tiefpassfilter mit einer Grenzfrequenz von ca. 10 kHz auf, womit ein Teil des Störschalles noch vor der A/D-Wandlung und der Signalanalyse herausgefiltert werden kann. Bei der gezeigten Messvorrichtung werden von Lautsprechern 2, 24 z.B. erzeugte Stimuli und/oder Suppressortöne über Kanäle und/oder Schläuche 6 zugeführt. Es ist jedoch auch möglich die Lautsprecher 2, 24, insbesondere wie in Fig. 1 gezeigt, direkt in der Sonde 1 anzuordnen. In einer einfachen Ausführung ist nur ein Lautsprecher 2 vorgesehen. Die Ausführung mit zwei Lautsprechern 2, oder drei für einen zusätzlichen Suppressorton, eignet sich speziell für die Messung von Distortionsprodukten, wie weiter unten noch anhand von Fig. 6 beschrieben wird. Die Stimuli und Suppressortöne werden von einem Signalgenerator 9 erzeugt. Der Druck zwischen Trommelfell und Gehörgangssonde kann mit einem Druckregulationssystem gesteuert werden. Dieses misst den Druck mittels eines Druckmessers 14 und stellt ihn mittels einer Druckpumpe 15 auf einen Sollwert ein. In der bevorzugten Ausführungsform ist der Raum zwischen Sonde und Innenohr mit Luft gefüllt. Es können auch andere Übertragungssubstanzen als Luft verwendet werden. Die mit dem Mikrophon 3 gemessenen otoakustischen Emissionen werden mit dem Computersystem 4 ausgewertet. Analoge Signale werden dazu digitalisiert. Bei Registrier- und Prüfmessungen wird jeweils, insbesondere mit Hilfe eines Analysemittels 10, aus den Messsignalen und gegebenenfalls den Messparametern ein biometrischer Datensatz abgeleitet. Bei Registriermessungen wird der Datensatz insbesondere zusammen mit dem Namen und/oder Kenndaten des Individuums in Speichermittel 11 abgelegt. Bei Prüfmessungen werden die Messsignale und/oder die daraus abgeleiteten Datensätze mit einem Vergleichsmittel 12 mit in Speichermittel 11 abgelegten Datensätzen verglichen. Die im Computersystem 4 gezeigten Blöcke 9, 10, 11, 12 und Verbindungslinien sind primär logischer Natur und müssen nicht als separate Hardwarebauteile bzw. Softwarekomponenten ausgeführt sein.

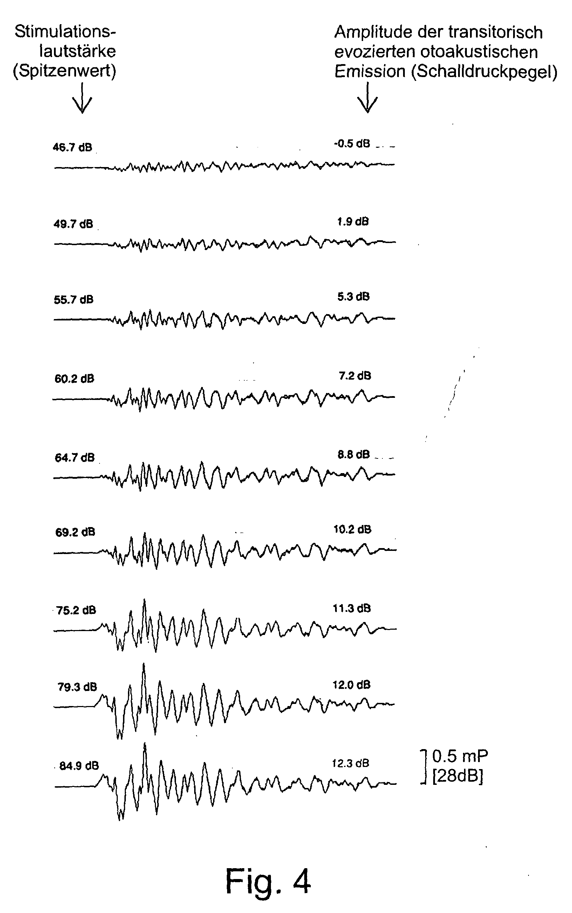

Fig. 4 zeigt Messsignale transitorischer klick-evozierter otoakustischer Emissionen eines Erwachsenen bei neun unterschiedlichen Stimuluslautstärken. Stimuli werden verwendet um otoakustische Emissionen zu evozieren. Der Stimulus kann als Klick, frequenzspezifischer Tone-Burst, diskreter Sinuston oder Sweep, d.h. in der Frequenz ansteigender oder absteigender Sinuston, oder als Kombination solcher Stimuli ausgestaltet sein, insbesondere im Frequenzbereich von 0.5 bis 10kHz. Dabei ist es wichtig zu betonen, dass die Anworten abhängig von den benutzten Stimuli mit einer gewissen Zeitverzögerung (Latenz) auch dann auftreten, wenn der Stimulationsschalldruck entsprechend den heute verfügbaren Messtechniken bereits wieder Null beträgt. Die gezeigten Antworten wurden durch Klicks mit unterschiedlichen Lautstärken akustisch evoziert. Bei den gezeigten Signalen wurde der Stimulus aus der Gesamtantwort eliminiert. Die Antwort auf die Evokation ist in einem Zeitfenster von 2.5 bis 20 ms, gemessen ab dem Ende des Stimulus, dargestellt. Bei der erfindungsgemässen Vorrichtung werden die Messwerte bevorzugt derart erfasst oder allenfalls gefiltert, dass der Satz von Messwerten für die Antwort auf einen Stimulus im Wesentlichen Messwerte in einem Zeitfenster ab ca. 2.5 ms und insbesondere bis ca. 20 ms oder bis ca. 100 ms nach Ende des Stimulus umfasst. Die Messwerte von 0 ms bis 2.5 ms nach Ende des Stimulus werden bewusst nicht erfasst. Während dieser Zeit dominiert das passive akustische Echo des Ohrs, welches bei der vorliegenden Erfindung im Wesentlichen nicht erfasst oder herausgefiltert wird. Das passive akustische Echo des Ohrs wird allenfalls separat erfasst und ausgewertet. Es ist auch möglich das passive akustische Echo des Ohrs separat zu messen, um es dann für die Bestimmung der otoakustischen Emissionen gezielt aus dem Signal herauszufiltern. Die Stimuli werden bevorzugt so leise gewählt, dass sie von der überwachten Person nicht wahrgenommen werden, oder zumindest nicht als störend empfunden werden. Insbesondere beim Einsatz in einer Umgebung mit kontinuierlichem Umgebungsschall, wie z.B. in einem Fahrzeug oder Flugzeug, können die akustischen Stimuli bezüglich Intensität und Spektrum so gewählt werden, dass sie aus Sicht der überwachten Person vom Umgebungsschall überdeckt sind und im Wesentlichen nicht wahrgenommen werden, was insbesondere für eine kontinuierliche Überwachung vorteilhaft ist. Umgebungsgeräusche können bei Bedarf auch durch Antischall zumindest teilweise ausgelöscht werden. Der Geräuschabstand der Messung kann dadurch verbessert werden. Die Messung kann auch, wie in Figur 4 gezeigt, in mehreren Messzyklen, unter Variation eines Parameters, in diesem Fall der Stimuluslautstärke, wiederholt werden. Das Verhalten der Amplitude ist bei steigender Stimulationslautstärke nichtlinear, d.h. die Amplitude nimmt bei geringeren Lautstärken relativ stärker zu als bei lauteren. Es zeigt sich ein sog. Sättigungseffekt, der als Parameter zur Analyse genutzt werden kann. Dabei zeigt sich, dass die Amplitudenanteile im höheren Frequenzbereich und/oder mit physikalisch kürzeren Laufzeiten relativ stärker zunehmen als die tieferen, die erst später im Messfenster auftreten. Die zur Identitätsüberprüfung zu analysierenden Bereiche können beliebig ausgewählt werden. Neben der Stimulationslautstärke können aber auch andere Messparameter variiert werden, z.B. die Typen der akustischen Stimuli und deren Kombination, die Stimulusfrequenz bzw. das Stimulusspektrum, die Dauer und Phase der Stimuli, die Anordnung der jeweiligen Stimulusgruppe, z.B. lineare oder nicht-lineare Stimulusgruppe, der Mittelohrdruck, die Spannung des Trommelfells und/oder der Innenohrdruck, oder es können zusätzliche sog. Suppressortöne eingeführt werden. Ein Suppressorton führt gewöhnlich zu einer Reduktion der Intensität der otoakustischen Emissionen. Durch eine systematische Variation der Messparameter können als biometrische Daten insbesondere auch oder im Wesentlichen Nichtlinearitäten ermittelt werden, wobei gezielt der linare- oder nichtlineare Anteil der Antwort bzw. Messung analysiert wird. Die Möglichkeit des nicht-linearen Stimulusarrangements und die Messung der verzögerten, aktiven, nicht-linearen cochleären Antwort kann zu einer hohen Genauigkeit des erfindungsgemässen Verfahrens bzw. Systems beitragen.

Fig. 5 zeigt die Messsignale bzw. die Wellenformen der transitorisch evozierten otoakustischen Emissionen (TEOAE) zweier Erwachsenenohren. Die'obere Graphik, welche die Messwerte einer ersten Person darstellt, unterscheidet sich alleine schon visuell von der zweiten Graphik, welche die Messwerte eine zweiten Person darstellt. Die Antworten sind im Zeitbereich 21 und Frequenzbereich 22 dargestellt. Die Darstellung im Frequenzbereich 22 umfasst neben der Antwort auch den Umgebungsschall, dessen Spektrum gegenüber dem grau dargestellten Spektrum der Antwort schwarz hervorgehoben ist. In beiden Fällen lässt sich unter sonst gleichen Messbedingungen eine hohe Reproduzierbarkeit (zwei superponierte Wellenkurven A bzw. B) hier in einem zeitlichen Messfenster von 2,5 bis ca. 20 ms nachweisen. Ein wesentlicher Unterschied zwischen den beiden Kurven der beiden Personen kann bereits visuell im Hinblick auf die unterschiedliche Gesamtamplitude der beiden TEOAE und der emittierten Frequenzanteile (jeweils Einschub oben rechts) und ihrer Amplitude nach Fourieranalyse erkannt werden. Die "sägezahnartigen" Wellenkurven können mit Schlüsseln für ein bestimmtes Schloss verglichen werden. Diese Wellenkurven sind für ein individuelles Ohr charakteristisch. Die Amplitude und Wellenform ist jedoch kein absolutes Bewertungskriterium und kann im Zusammenhang mit anderen Messwerten auch abschnittsweise analysiert werden.

Fig. 6 zeigt ein Messsignal mit Stimuli und otoakustischer Antwort im Frequenzbereich. Bei der gezeigten Messung wird eine Distortionsproduktemission 25 gemessen. Dabei wird der Effekt ausgenützt, dass zwei Stimuli 23, einer bei einer niederen Frequenz f₁ und einer bei einer höheren Frequenz f₂, als Antwort eine otoakustische Emission bei einer dritten Frequenz 2f₁-f₂ evozieren. Die Antwort wird von der erfindungsgemässen Vorrichtung gezielt bei der dritten Frequenz gemessen, z.B. mittels einer geeigneten Bandpassfilterung, d.h. es werden insbesondere Messdaten ermittelt, welche eine Antwort bei der dritten Frequenz beschreiben. Insbesondere werden dabei die Stimulustöne herausgefiltert. Die beiden diskreten Töne des Stimuli 23 werden bevorzugt, wie im Blockdiagramm von Figur 3 gezeigt, von zwei separaten Lautsprechern erzeugt. Die Erzeugung der beiden Töne mit nur einem Lautsprecher ist auch möglich, hat jedoch den Nachteil, dass die Distortionscharakteristik des Lautsprechers in das Messresultat mit eingeht. Beispiele für Frequenzpaare sind: f₁=430 Hz, f₂=581 Hz, f₁=1789 Hz, f₂=2236 Hz und f₁=7460 Hz, f₂=8579 Hz. Neben der Frequenz kann auch die Lautstärke L₁, L₂ der beiden Töne, insbesondere relativ zueinander, variiert werden. Bevorzugt wird die Differenz L₂-L₁ im Bereich von 6 bis 10 dB gewählt.

In einer bevorzugten Ausführung des erfindungsmässen Verfahrens werden bei der Registriermessung mehrere Messungen für eine bestimmte Auswahl verschiedener Messparameter und insbesondere verschiedener Stimuli vorgenommen. Für jedes registrierte Individuum wird so eine Bibliothek von auf jeweils verschiedenen Messparameterkonfigurationen basierenden Datensätzen angelegt. Bei der Prüfmessung wird dann jeweils einer dieser Datensätze bzw. dessen Messparameterkonfigurationen verwendet. Dies hat den Vorteil, dass ähnlich wie bei einem "Challenge-Response" Verfahren, Einmal-Passwörtern oder "Streichlisten" keine einfachen "Replay" Angriffe möglich sind. Es ist also nicht möglich die Prüfvorrichtung durch das Abspielen einer zuvor, d.h. bei einer beliebigen früheren Prüfmessung, aufgenommenen Antwort zu täuschen. Eine besonders hohe Sicherheit kann erreicht werden, wenn bei jeder Prüfmessung ein zuvor nicht benutzter Datensatz bzw. dessen Messparameterkonfiguration verwendet wird. Dies hat jedoch den Nachteil, dass die Anzahl der so ausführbaren Prüfmessungen begrenzt ist. Die Sicherheit kann zusätzlich verbessert werden, indem bei jeder Prüfmessung. der Datensatz und damit eine bestimmte Messparameterkonfiguration jeweils im Wesentlichen zufällig ausgewählt wird. Damit ist die Messparameterkonfiguration selbst bei. Kenntnis des Systems und seines Zustandes nicht vorhersagbar, was das Vortäuschen einer korrekten Antwort erschwert bzw. praktisch verunmöglicht.

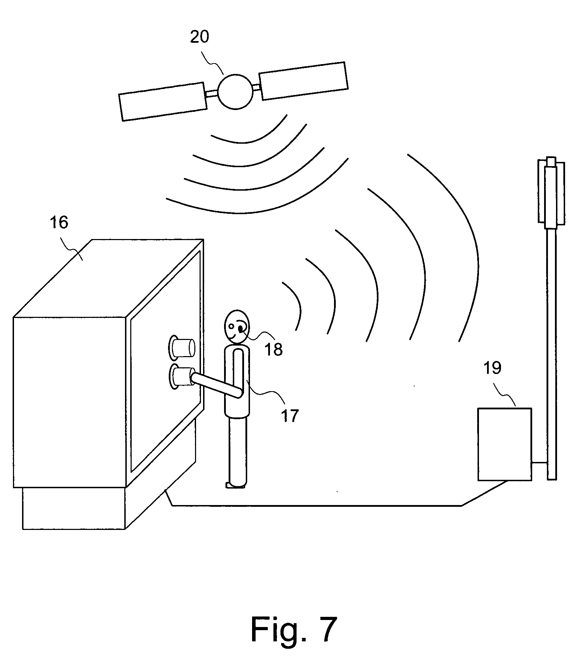

Fig. 7 zeigt ein Anwendungsbeispiel des erfindungsgemässen Verfahrens zur Sicherung eines Tresorraumes bzw. eines Tresors 16. Ziel dieser Anwendung ist es zu verhindern, dass eine nicht autorisierte Person unerkannt anstelle einer autorisierten Person in einem Tresorraum arbeiten kann. Das gezeigte System umfasst eine bevorzugte Ausführung der erfindungsgemässen mobilen Überwachungsvorrichtung 18 und eine Basisstation 19. Die mobile und insbesondere tragbare Überwachungsvorrichtung 18 wird von der autorisierten Person 17 wie ein Hörgerät im Ohr getragen und ermöglicht zu jedem Zeitpunkt eine Identitäts- und inbesondere Vitalitätsüberprüfung. Diese wird bevorzugt in Intervallen von einigen Sekunden bis Minuten ausgeführt und ist damit im Wesentlichen kontinuierlich durchführbar. Der kontinuierliche Identifikations- und Überprüfungsprozess gemäss der Erfindung erlaubt zudem den simultanen Nachweis intellektueller und/oder physischer Zuverlässigkeit. Die tragbare Überwachungsvorrichtung 18 kommuniziert über Funk mit der Basisstation 19. Die Messresultate können entweder im Wesentlichen "online" während der Messung übermittelt werden, oder "offline" nach der Messung. Die Kommunikation erfolgt vorzugsweise verschlüsselt und es sind dem Fachmann bekannte Verfahren vorgesehen um die tragbare Überwachungsvorrichtung 18 gegenüber der Basisstation 19 zu authentisieren. Die tragbare Überwachungsvorrichtung 18 hat zudem vorzugsweise ein Mittel zur Postitionsbestimmung, insbesondere satellitengestützt, basierend auf GPS (Global Positioning System), wobei das Mittel zur Postitionsbestimmung Signale von Satelliten 20 empfängt. Falls nun das System keine autorisierte Person erkennt, oder die Person 19 den Bereich verlässt, für den sie autorisiert ist, wird ein Alarm ausgelöst und/oder Zugangsbarrieren, z.B. eine Tresortüre, gesperrt. In einer ersten Ausführungsform ist die tragbare Überwachungsvorrichtung 18 im Wesentlichen als drahtlos mit einer Basisstation 19 kommunizierende Sonde ausgestaltet, d.h. Stimulusgeneration und Messwertanalyse erfolgen in der Basisstation 19. In einer zweiten Ausführungsform erlaubt die tragbare Überwachungsvorrichtung 18 ohne Kommunikation mit der Basisstation 19 die Ausführung des erfindungsgemässen Verfahrens zur Identitätsüberprüfung bzw. Erkennung und insbesondere auch Registrierung, wobei die wesentlichen Systemkomponenten, insbesondere die Mittel zum Analysieren, Vergleichen und Speichern biometrischer Daten, in der tragbaren Überwachungsvorrichtung 18 integriert sein können. Die tragbare Überwachungsvorrichtung 18 kommuniziert in diesem Fall mit der Basisstation 19 nur noch zur Übermittlung eines Autorisierungssignals, jedoch ohne dass biometrische Daten selbst übermittelt werden. Neben den zwei oben beschriebenen Ausführungsformen können auch je nach Anforderungen an das System die verschiedenen Verfahrensschritte bzw. Systemkomponenten in vielfältiger Weise auf die mobile Überwachunsvorrichtung 18, die Basisstation 19 und allfällige weitere Einheiten verteilt werden. Die tragbare Überwachungsvorrichtung 18 kann vorzugsweise als Im-Ohr-Gerät, Hinter-dem-Ohr-Gerät, Stirnbandgerät oder Tragegurtgerät ausgestaltet sein. Die Kommunikation mit der Basisstation kann über ein öffentliches Mobilfunknetz erfolgen, wobei die tragbare Überwachungsvorrichtung 18 dann insbesondere über eine Schnittstelle zur Kommunikation mit einem handelsüblichen Mobiltelefon verfügt. Des Weiteren kann die Vorrichtung selbst zum Empfang und/oder zur Übermittlung von Sprachsignalen ausgestaltet sein, wobei im Wesentlichen die Funktion eines Mobiltelefons in die Vorrichtung integriert wird. Auf diese Weise können über das System Handlungsaufforderungen und/oder Anweisungen an die Person 17 zur Ausführung spezifischer Aufgaben weitergegeben werden.

Das erfindungsgemässe Verfahren kann also unter anderem zu Identifizierungs- und Verifizierungzwekken, zur Sicherheitsüberprüfung beim Zugang zu sensiblen Daten oder Sicherheitsbereichen, zu Überwachungszwecken und zur Aktivierung oder Deaktivierung von Systemen oder Systemteilen, wie z.B. Software und Hardware von Computersystemen, aber auch sonstigen Maschinen oder Prozessabläufen eingesetzt werden. Das erfindungsgemässe Verfahren kann insbesondere auch für den Gebrauch einer Software definiert werden, so dass jederzeit für den Eigenbedarf Softwarekopien gemacht werden können, jedoch beim Gebrauch der Software die Nutzung durch den individuellen Nutzer anhand der individuellen Innenohraktivität abgefragt wird. Die oben beschriebenen Anwendungen sind, wie bereits anhand von Fig. 7 gezeigt, nicht räumlich gebunden und beschränkt auf eine bestimmte Position im geografischen Koordinatensystem. Sie können vorteilhaft und typischerweise auch über jegliche Technologie zur Datenfernübertragung genutzt werden, wie z.B. über das Internet, Funk, optische Übertragung etc. In Verbindung mit GPS können z.B. auch Vorrichtungen für den elektronisch überwachten Hausarrest als preisgünstige Alternative zum Strafvollzug in Strafanstalten realisiert werden.

Wie bereits angedeutet kann das erfindungsgemässe Verfahren mit bekannten, insbesondere biometrischen Autorisierungsverfahren kombiniert werden. So kann z.B. zusätzlich ein Geheimcode oder ein Dongle bzw. Token bereitgestellt werden. Die verschiedenen Verfahren können entweder hintereinandergeschaltet werden, um eine höhere Sicherheit zu erreichen, oder aber nebeneinander eingesetzt werden, um durch eine Redundanz der Zugangswege eine höhere Zugangszuverlässigkeit zu erreichen. Durch das sequentielle, d.h. hintereinandergeschaltete, Ausführen mehrerer Messungen bei unterschiedlichen Messparametern lässt sich insbesondere eine Vielzahl verschiedener Sicherheitsstufen realisieren.

## Patentansprüche

1. Verfahren zur Registrierung und späteren Erkennung und/oder Überprüfung einer Identität eines Individuums (17), wobei von mindestens einem Individuum (17) mittels einer Registriermessung erste biometrische Daten ermittelt werden und zur Erkennung und/oder Überprüfung der Identität eines Individuums (17) mittels einer Prüfmessung zweite biometrische Daten ermittelt werden und die ersten biometrischen Daten mit den zweiten biometrischen Daten verglichen werden, **dadurch gekennzeichnet, dass** die Registriermessung und die Prüfmessung jeweils eine Messung otoakustischer Emissionen umfassen.

2. Verfahren gemäss Anspruch 1, **dadurch gekennzeichnet, dass** die otoakustischen Emissionen direkt im Ohr gemessen werden, insbesondere mittels einer Gehörgangssonde (1) in einem Bereich vor einem Trommelfell oder mittels eines Implantates in einem Bereich hinter einem Trommelfell.

3. Verfahren gemäss einem der vorhergehenden Ansprüche, **dadurch gekennzeichnet, dass** die otoakustischen Emissionen mittels mindestens eines Stimulus akustisch evoziert werden und eine Antwort auf eine akustische Evokation gemessen wird und insbesondere, dass der Stimulus mindestens ein Signal aus der Gruppe umfassend Klicks, Bursts, diskrete Sinustöne und Sweeps umfasst, insbesondere im Bereich von 0.5 bis 10kHz.

4. Verfahren gemäss Anspruch 3 zum Einsatz in einer Umgebung mit kontinuierlichem Umgebungsschall, **dadurch gekennzeichnet, dass** der Stimulus bezüglich seiner Intensität und/oder seines Frequenzspektrums derart gewählt wird, dass er aus der Sicht des Individuums im Wesentlichen vom Umgebungsschall abgedeckt ist.

5. Verfahren gemäss einem der Ansprüche 3 oder 4, **dadurch gekennzeichnet, dass** Messwerte derart erfasst und/oder gefiltert werten, dass ein Satz von Messwerten im Wesentlichen nur Messwerte in einem Zeitfenster ab ca. 2.5 ms und insbesondere bis ca. 20 ms oder bis ca. 100 ms nach Ende des Stimulus umfasst und/oder ein passives Echo des Stimulus im Wesentlichen nicht erfasst oder herausgefiltert wird.

6. Verfahren gemäss einem der Ansprüche 3 bis 5, **dadurch gekennzeichnet, dass** der Stimulus im Wesentlichen einen Ton bei einer ersten Frequenz f₁ und einen Ton bei einer zweiten Frequenz f₂ umfasst und Messdaten ermittelt werden, welche eine Antwort bei einer Frequenz *i·f*_{*1*}*+j·f*_{*2*} mit *i, j ∈* Z beschreiben, wobei insbesondere *i = 2* und *j*=-1.

7. Verfahren gemäss einem der vorhergehenden Ansprüche, **dadurch gekennzeichnet, dass** während der Messung otoakustischer Emissionen zusätzlich ein Suppressorton erzeugt wird.

8. Verfahren gemäss einem der vorhergehenden Ansprüche, **dadurch gekennzeichnet, dass** die Messung otoakustischer Emissionen eine Vielzahl von Messzyklen umfasst, insbesondere eine Vielzahl von Messzyklen, bei der mittels Parametervariation Nichtlinearitäten erfasst werden.

9. Verfahren gemäss einem der vorhergehenden Ansprüche, **dadurch gekennzeichnet, dass** ein Druck zwischen einer Gehörgangssonde (1) und einem Trommelfell vor und/oder während der Messung otoakustischer Emissionen gemessen, geregelt und/oder gesteuert wird.

10. Verfahren gemäss einem der vorhergehenden Ansprüche, **dadurch gekennzeichnet, dass** die ersten biometrischen Daten und die zweiten biometrischen Daten in einem Frequenzbereich vorliegende und/oder in einen Frequenzbereich transformierbare Signaldaten aufweisen und Signaldaten der ersten biometrischen Daten mit Signaldaten der zweiten biometrischen Daten im Frequenzbereich verglichen werden.

11. Verfahren gemäss einem der vorhergehenden Ansprüche, **dadurch gekennzeichnet, dass** die Registriermessung mehrere Messungen basierend auf einer Auswahl verschiedener Messparameterkonfigurationen umfasst und insbesondere bei einer Prüfmessung jeweils eine zufällig gewählte und/oder in vorangehenden Prüfmessungen noch nicht verwendete Messparameterkonfiguration aus dieser Auswahl verwendet wird.

12. Verfahren gemäss einem der vorhergehenden Ansprüche, **dadurch gekennzeichnet, dass** während der Messung otoakustischer Emissionen ein Antischall erzeugt wird, insbesondere zur Verbesserung eines Geräuschabstandes durch eine zumindest teilweisen Auslöschung von Umgebungsgeräuschen.

13. Vorrichtung zur Ermittlung biometrischer Daten eines Individuums (17), insbesondere zur Anwendung in einem Verfahren gemäss einem der Ansprüche 1 bis 12, **dadurch gekennzeichnet, dass** sie ein Mittel zur Messung otoakustischer Emissionen aufweist.

14. Vorrichtung gemäss Anspruch 13, **dadurch gekennzeichnet, dass** sie ein Mittel zur Erzeugung eines Stimulus und/oder Suppressortons aufweist, insbesondere umfassend ein, zwei oder drei Lautsprecher (2, 24).

15. Vorrichtung gemäss einem der Ansprüche 13 oder 14, wobei die Vorrichtung eine Gehörgangssonde (1) aufweist, **dadurch gekennzeichnet, dass** sie Mittel zur Messung und insbesondere Steuerung eines Druckes zwischen der Gehörgangssonde (1) und einem Trommelfell aufweist, insbesondere einen Drucksensor (14) und eine Pumpe (15), welche über einen Schlauch und/oder Kanal mit der Gehörgangssonde (1) verbunden ist.

16. Mobile Überwachungsvorrichtung zur Überwachung eines Individuums (17), **dadurch gekennzeichnet, dass** sie eine Vorrichtung gemäss einem der Ansprüche 13 bis 15, und ein Mittel zur Kommunikation mit einer Basisstation (19) aufweist, insbesondere ein Mittel zur drahtlosen Kommunikation mit einer Basisstation (19).

17. Mobile Überwachungsvorrichtung gemäss Anspruch 16, **dadurch gekennzeichnet, dass** sie ein Mittel zur Positionsbestimmung aufweist, insbesondere ein Mittel zur satellitengestützten Positionsbestimmung.

18. Mobile Überwachungsvorrichtung gemäss einem der Ansprüche 16 oder 17, **dadurch gekennzeichnet, dass** sie Mittel zum Empfang und zur Wiedergabe von Sprachsignalen aufweist und/oder Mittel zur Aufnahme und Übermittlung von Sprachsignalen aufweist und insbesondere als Mobiltelefon verwendbar ist.

19. System zur Registrierung und späteren Erkennung und/oder Überprüfung einer Identität eines Individuums (17), insbesondere zur Ausführung eines Verfahrens von einem der Ansprüche 1 bis 12, **dadurch gekennzeichnet, dass** es ein Mittel (11) zur Speicherung biometrischer Daten, ein Mittel (12) zum Vergleich biometrischer Daten und eine Vorrichtung gemäss einem der Ansprüche 12 bis 17 aufweist.

20. System zur Sicherung eines Zugangs zu einem virtuellen und/oder physikalischen geschützten Bereich, **dadurch gekennzeichnet, dass** es ein System zur Registrierung und späteren Erkennung und/oder Überprüfung der Identität eines Individuums (17) gemäss Anspruch 19 und mindestens ein Mittel zur Gewährung und/oder Sperrung eines Zuganges zu dem geschützten Bereich oder ein Mittel zum Auslösen eines Alarms in Abhängigkeit eines Resultates der Erkennung und/oder Überprüfung aufweist.
